# FASCICULE DE BREVET EUROPEEN

(11) **EP 1 991 519 B1**
(45) Date de publication et mention de la délivrance du brevet: **26.05.2010**
(21) Numéro de dépôt: 07731070.4
(22) Date de dépôt: 02.03.2007
(51) Int. Cl.: C07C 69/34, C07C 67/22, C08K 5/11

(54) **DIESTERS D'ACIDES CARBOXYLIQUE RAMIFIES**
VERZWEIGTE DICARBONSÄURE-DIESTER
BRANCHED CARBOXYLIC ACID DIESTERS

(30) Priorité: 07.03.2006 FR 0602011
(43) Date de publication de la demande: 19.11.2008
(73) Titulaire: Rhodia Opérations, 93306 Aubervilliers (FR)
(72) Inventeur: MARION, Philippe, F-69390 VERNAISON (FR); TROUILLET-FONTI, Lise, F-69003 Lyon (FR); BERNARD, Jean-Marie, F-69440 SAINT-LAURENT D'AGNY (FR)
(74) Mandataire: Boittiaux, Vincent
(86) Numéro de dépôt international: PCT/FR2007/000370
(87) Numéro de publication internationale: WO 2007/101929

(56) Documents cités:
- EP-A1- 0 055 875
- DE-A1- 2 950 100
- US-A- 2 801 263
- US-A- 3 567 749
- MATSUDA, AKIO: "Cobalt carbonyl-catalyzed hydroesterification of butadiene with carbon monoxide and methanol" BULLETIN OF THE CHEMICAL SOCIETY OF JAPAN , 46(2), 524-30 CODEN: BCSJA8; ISSN: 0009-2673, 1973, XP002407465
- MARKETTA U.-P. ET AL: "Liquid-Liquid Equilibria of Selected Dibasic Ester + Water + Solvent Ternary Systems" JOURNAL OF CHEMICAL AND ENGINEERING DATA, AMERICAN CHEMICAL SOCIETY, US, vol. 41, 1996, pages 235-238, XP002407466 ISSN: 0021-9568

## Description

La présente invention concerne des diesters d'acides carboxyliques présentant notamment des propriétés de solvatation intéressantes et pouvant être utilisés en substitution de solvants usuels notamment halogénés.

L'invention concerne plus particulièrement une composition de diesters d'acides carboxyliques ramifiés obtenus à partir d'un mélange de dinitriles. Ce mélange de dinitriles est obtenu comme fraction de distillation au cours de la récupération et purification de l'adiponitrile dans le procédé de fabrication de ce dernier composé par double hydrocyanation du butadiène.

Depuis plusieurs années, un solvant oxygéné est proposé. Ce solvant est à base de diesters obtenus par estérification d'un mélange de diacides carboxyliques, plus particulièrement d'un mélange d'acide adipique, glutarique et succinique. Ce mélange d'acides est obtenu dans le procédé de fabrication de l'acide adipique par oxydation de cyclohexanol et/ou cyclohexanone.

Ce solvant commercialisé, par exemple, par la société Rhodia sous le nom commercial RHODIASOLV RPDE et par la société Invista sous l'appellation commerciale DBE est utilisé dans de nombreuses applications, notamment en remplacement de solvants hydrocarbonés, solvants chlorés ou solvants oxygénés (glycols éthers ; acétone).
En plus de ces performances techniques conséquences de son pouvoir solvant et de ses propriétés physico-chimiques, ce solvant oxygéné présente l'avantage d'être moins nocif pour l'environnement, biodégradable et facilement recyclable. Son profil toxicologique très favorable, permet de supprimer tous les risques pour l'utilisateur final. En outre, les propriétés physico-chimiques telles que la faible volatilité et le point éclair permettent une utilisation en toute sécurité.

Il peut également être utilisé en mélange avec d'autres solvants comme la N-méthyl pyrrolidone (NMP) sans affecter les propriétés solvant mais en diminuant le coût de celui-ci.

De plus, ce solvant est stable à température ambiante et présente une faible tension de vapeur.

Les solvants oxygénés présentant de bonnes propriétés de solvatation et sans risque de toxicité et sans danger pour l'environnement connaissent un très fort développement. Il est donc important pour le domaine de l'industrie de trouver et proposer de nouveaux solvants présentant des propriétés au moins similaires ou équivalentes à celles des solvants déjà disponibles tels que le RPDE cité ci-dessus.
Le document "Liquid-Liquid Equilibria of Selected Dibasic Ester + Water + Solvent Ternary Systems", J. Chem. Eng. Data 1996, 41, 235-238, décrit un mélange de diesters d'isobutylol et d'un mélange d'acide succinique, d'acide glutarique et d'acide adipique (produit "DBE" dans le document). Il décrit également les propriétés de mélanges comprenant un alcool, de l'eau et des diesters comme le produit "DBE".
Un des buts de la présente invention est de proposer un nouveau solvant oxygéné présentant des propriétés physiques, de solvant, un profil toxicologique et un impact écotoxique semblables ou améliorées par rapport au solvant à base de diesters d'un mélange d'acide adipique, glutarique et succinique.

A cet effet, l'invention propose l'utilisation à titre de solvant ou co-solvant d'une composition comprenant un mélange de diesters d'acide éthylsuccinique, méthylglutarique et éventuellement acide adipique.

Selon une autre caractéristique de l'invention, ce mélange contient :
➢ De 70 à 95% en poids de diesters d'acide méthylglutarique
➢ De 5 à 30% en poids de diesters d'acide ethylsuccinique
➢ De 0 à 10% en poids de diesters d'acide adipique

L'invention propose également un composition particulière, comprenant un mélange de diesters d'acide éthylsuccinique, d'acide méthylglutarique et d'acide adipique, caractérisée en ce que la concentration pondérale de ses différents composants est :
➢ Diesters de l'acide méthylglutarique comprise entre 70 et 95%
➢ Diesters de l'acide éthylsuccinique comprise entre 5 et 30%
➢ Diesters de l'acide adipique iusqu'à 10%.

Cette composition est obtenue à partir d'un mélange de composés dinitriles notamment produit et récupéré dans le procédé de fabrication de l'adiponitrile par double hydrocyanation du butadiène.

Ce procédé utilisé à grande échelle dans l'industrie pour produire la grande majorité de l'adiponitrile consommé dans le monde est décrit dans de nombreux brevets et ouvrages.

La réaction d'hydrocyanation du butadiène conduit majoritairement à la formation de dinitriles linéaires mais également à une formation de dinitriles ramifiés dont les deux principaux sont le méthylglutaronitrile et l'ethylsuccinonitrile.

Dans les étapes de séparation et purification de l'adiponitrile, les composé dinitriles ramifiés sont séparés par distillation et récupérés, par exemple, comme fraction de tête dans une colonne de distillation.

L'invention propose de transformer ce mélange de composés dinitriles ramifiés en diesters pour ainsi obtenir un nouveau solvant.

Il peut être intéressant d'éliminer du mélange de dinitriles ramifiés récupérés, les produits plus volatils par une simple distillation, par exemple.

Un des procédés possibles de transformation des composés dinitriles en diesters correspond à la mise en oeuvre de la réaction de PINNER, notamment décrite dans le brevet français n°1488857. Sommairement, ce procédé consiste à faire réagir les composés dinitriles avec un alcool en présence d'un acide minéral fort tel que l'acide sulfurique, puis à hydrolyser les produits obtenus pour récupérer les diesters par distillation.

Ce document décrit également un mode de réalisation particulier du procédé qui consiste à faire passer le mélange de composés dinitriles et l'alcool dans un bain de sels fondus à base de différents sulfates alcalins et ammonium pour éviter la formation de sulfate d'ammonium et récupérer l'ammoniaque par extraction à la vapeur d'eau.

Les diesters de l'invention peuvent également être obtenus par une réaction entre les composés dinitriles, l'eau et un alcool en phase gaz et en présence d'un catalyseur solide. La température de réaction est avantageusement supérieure à la température de condensation des diesters formés. Comme catalyseur, on peut utiliser un catalyseur solide acide tel que, par exemple, un gel de silice, un mélange silice-alumine, des zéplithes, la zircone, des acides boriques ou phosphoriques supportés. On peut également utiliser des alumines macroporeuses telles que celles décrites dans le brevet européen EP0805801.

La température de la réaction est comprise entre 200 et 450°C, de préférence entre 230 et 350°C. la réaction peut être réalisée sous une pression quelconque, avantageusement comprise entre 0,1 et 20 bar. En sortie de réacteur, les vapeurs sont refroidies rapidement à une température inférieure ou égale à 150°C. Du mélange obtenu, on sépare par distillation l'ammoniac puis l'eau et l'alcool en excès.

Les diesters de l'invention peuvent également être obtenus par réaction entre les composés dinitriles et une base minérale pour obtenir des sels d'acides, puis neutralisation de ces sels par un acide suivi d'une estérification avec un alcool. Les sels de diacides et notamment le sel d'ammonium des diacides peuvent être obtenus par hydrolyse enzymatique des composés nitriles tels que décrits par exemple dans les brevets EP5986812 et FR2700777.

Enfin, les diesters sont purifiés selon les procédés de purification classiquement utilisés dans le domaine technique de la préparation de composés organiques et notamment par distillation dans une ou plusieurs colonnes.

Selon l'invention, le mélange de diesters conforme à l'invention présente des propriétés particulières et différentes des propriétés du mélange de diesters obtenu par estérification d'acides carboxyliques linéaires.
Plus particulièrement, il présente une température de cristallisation inférieure à - 50°C ce qui permet une utilisation dans un domaine de température très large avec une viscosité du solvant faible.

A titre de comparaison, le solvant issu du mélange d'acides carboxyliques linéaires a une température de cristallisation comprise entre -20°C et +20°C selon leur composition.

La composition de l'invention présente également une faible solubilité dans l'eau. Egalement la solubilité de l'eau dans la composition est inférieure ou égale à 2,5% en poids à 23°C.

Les diesters formant la composition de l'invention sont obtenus par réaction d'un alcool avec les composés dinitriles cités précédemment. Comme alcool pouvant être utilisé pour la fabrication de ces composés, on peut citer les alcools aliphatiques ramifiés ou non, cycliques ou non, pouvant comporter un noyau aromatique et pouvant comprendre de 1 à 20 atomes de carbone. A titre d'exemples préférés, on peut citer les alcools suivants, méthanol, propanol, isopropanol, alcool benzylique, éthanol, n-butanol, isobutanol, pntanols, cyclohexanol, hexanol, isooctanol, 2-éthyl hexanol.

La composition de l'invention peut être utilisée seule ou en mélange avec d'autres solvants ou avec de l'eau sous forme de solution ou émulsion. Notamment, elles peuvent être utilisées en mélange avec les diesters des diacides linéaires cités précédemment.

Ces compositions trouvent des applications comme solvant dans de nombreux domaines tels que les peintures, vernis et laques, l'industrie du revêtement de surfaces ou de tout autre article comme les câbles par exemple, l'industrie des encres, des lubrifiants pour textiles, les liants et résines pour noyaux et moules de fonderie, des produits nettoyants, les formulations cosmétiques, pour la mise en oeuvre de certaines réactions chimiques, dans les compositions de traitement des sols et végétaux et plus généralement, l'utilisation seul ou dans une formulation, comme solvant de nettoyage, décapage, dégraissage dans toute activité industrielle ou domestique.
Ces compositions peuvent également être utilisées comme plastifiants de certaines matières plastiques ou comme monomères pour la fabrication de polymères.

D'autres avantages, caractéristiques de l'invention seront mieux détaillés et illustrés au vu des exemples donnés ci-après uniquement à titre illustratif.

### Exemple

Dans un réacteur en verre de contenance 500 ml muni d'un réfrigérant ascendant, d'un agitateur et chauffé par un bain d'huile, 43,26g d'un mélange A de composés dinitriles sont chargés avec 76,90g de méthanol.
Le mélange A de composés dinitriles est constitué de :
➢ 86,9 % en poids de méthylglutaronitrile
➢ 11,2 % en poids d'éthylsuccinonitrile
➢ 1,9 % en poids d'adiponitrile.

Le complément à 100% correspond aux impuretés présentes dans ce mélange qui ne sont généralement pas des composés dinitriles.
Le mélange composés dinitriles/méthanol est refroidi à environ 1 °C avant l'addition de 84,22 g d'acide sulfurique à 98% en poids.
Le milieu réactionnel est chauffé jusqu'à reflux et maintenu à cette température pendant 3h. La masse réactionnelle est hétérogène et fluide. Après refroidissement à 60°C, 63g d'eau sont ajoutés. Le milieu réactionnel est maintenu à 65°C pendant 2 heures.
On ajoute alors 117g d'eau supplémentaire. Le milieu réactionnel devient biphasique. Après élimination du méthanol en excès par évaporation, les deux phases sont décantées et analysées. La phase organique récupérée est lavée par une solution aqueuse saturée en chlorure de sodium avec addition d'ammoniaque pour obtenir un pH voisin de 7.

Un second lavage est effectué avec une solution aqueuse saturée en chlorure de sodium.

Après distillation de la phase organique lavée on obtient un mélange de composition suivante :

| | |
|---|---|
| Diméthyl 2 - méthylglutarate | : 89°% |
| Diméthyl 2 - ethylsuccinate | : 9% |
| Diméthyl adipate | : 1% |
| Divers composés | : 1% |

Ce mélange présente les propriétés suivantes :
➢ Température de cristallisation, (la détermination du point de cristallisation a été effectuée par utilisation de la méthode décrite dans la nonne NFT 20-051): inférieure à -50°C
➢ Solubilité avec l'eau :
   o Solubilité de l'eau dans la composition : 2,2 % en poids
   o Solubilité de la composition dans l'eau : 2,5% en poids

Cette valeur a été déterminée en utilisant un récipient muni d'un agitateur à une température de 23°C soit par addition d'eau dans 20 g de composition diesters, soit par addition de la composition diesters dans 50 g d'eau jusqu'à l'obtention d'une solution trouble.

### Résistance à l'hydrolyse :

L'acidité est réalisée en ajoutant 5 g de composition diesters dans 95g d'eau contenant 8 millimoles de NaOH. le flacon est disposé pendant plusieurs jours dans une enceinte chauffée à 50°C.

L'acidité du milieu est mesurée périodiquement pour contrôler l'abaissement du pH.
Ces tests ont été réalisés avec la composition diesters de l'invention et à titre comparatif avec une composition diesters issus d'un mélange de diacides linéaires commercialisée par la société Rhodia sous l'appellation commerciale RPDE.

Ils démontrent que la composition de l'invention est plus résistante à l'hydrolyse que la composition RPDE.

De même des essais comparatifs ont été réalisés pour évaluer le pouvoir de solvatation des diesters de l'invention par rapport à celui de la composition RPDE de la société RHODIA.

Ces essais ont été conduits en mélangeant dans un récipient des résines habituellement employées dans le domaine de la peinture avec une quantité déterminée de solvant diesters ou RPDE.

Les résultats observés sont listés dans les tableaux I et II ci-dessous :

**TAB I**

| Solvant RPDE % en poids | *10%* | *20%* | *30 %* | *40%* | 50% |
|---|---|---|---|---|---|
| Résines | | | | | |
| Epoxy Epikote 828 Commercialisée par RESOLUTION PERFORMANCE PRODUCT | Soluble | soluble (très léger trouble après 2 jours) | soluble (léger trouble après 2 jours) | soluble (léger trouble après 2 jours) | Soluble |
| IsocyanateTolonate HDT Commercialisée par RHODIA | Soluble | Soluble | Soluble | | Soluble |
| Alkyde Coporob 2526 Commercialisée par NOVANCE | Soluble | Soluble | | | Soluble |
| Alkyde Coporob 335 60 Commercialisée par NOVANCE | Soluble | Soluble | | | Soluble |
| Polyacrylique polyol Macrynal SM516 Commercialisée par SOLUTIA | Soluble | Soluble | | | Soluble |

**TAB II**

| Diesters de l'invention % en poids | *10%* | *20%* | *30%* | *40%* | *50%* |
|---|---|---|---|---|---|
| Résines | | | | | |
| Epoxy Epikote 828 | Soluble | soluble (très léger trouble après 2 jours) | soluble (léger trouble après 2 jours) | soluble (léger trouble après 2 jours) | Soluble |
| Isocyanate Tolonate HDT | Soluble | Soluble | Soluble | | Soluble |
| Alkyde Coporob 2526 | Soluble | Soluble | | | Soluble |
| Alkyde Coporob 335 60 | Soluble | Soluble | | | Soluble |
| Polyacrylique polyol Macrynal SM516 | Soluble | Soluble | | | Soluble |

Les essais ci-dessus montrent que le pouvoir solvant des diesters de l'invention est au moins équivalent à celui du solvant RPDE.

## Revendications

1. Utilisation comme solvant ou co-solvant d'une composition comprenant un mélange de diesters d'acide éthylsuccinique, et d'acide méthylglutarique.

2. Utilisation selon la revendication 1 **caractérisée en ce que** la composition comprend des diesters de l'acide adipique.

3. Utilisation selon l'une des revendications 1 ou 2, **caractérisée en ce que** la concentration pondérale de ses différents composants de la composition est :
➢ Diesters de l'acide méthylglutarique comprise entre 70 et 95%
➢ Diesters de l'acide éthylsuccinique comprise entre 5 et 30%
➢ Diesters de l'acide adipique comprise entre 0 et 10%

4. Utilisation selon l'une des revendications précédentes **caractérisée en ce que** la composition présente une température de cristallisation inférieure à -50°C

5. Utilisation selon l'une des revendications précédentes **caractérisée en ce que** la solubilité de l'eau dans ladite composition est inférieure ou égale à 2,5% en poids à 23°C

6. Utilisation selon l'une des revendications précédentes **caractérisée en ce que** la composition est obtenue par estérification de composés dinitriles correspondants.

7. Utilisation selon la revendication 6, **caractérisée en ce que** les composés dinitriles utilisés sont présents dans le mélange des dinitriles ramifiés séparés de l'adiponitrile dans le procédé de fabrication de l'adiponitrile par double hydrocyanation du butadiène.

8. Utilisation selon l'une des revendications précédentes, **caractérisée en ce que** les diesters sont obtenus par réaction d'un composé dinitrile avec un alcool choisi dans le groupe comprenant les alcools aliphatiques ramifiés ou non, cycliques ou non, pouvant comporter un noyau aromatique et pouvant comprendre de 1 à 20 atomes de carbone.

9. Utilisation selon la revendication 8, **caractérisée en ce que** l'alcool est choisi dans le groupe comprenant méthanol, propanol, isopropanol, alcool benzylique, éthanol, n-butanol, isobutanol, pentanols, cyclohexanol, hexanol, isooctanol, 2-éthyl hexanol.

10. Composition comprenant un mélange de diesters d'acide éthylsuccinique, d'acide méthylglutarique et d'acide adipique, **caractérisée en ce que** la concentration pondérale de ses différents composants est :
➢ Diesters de l'acide méthylglutarique comprise entre 70 et 95%
➢ Diesters de l'acide éthylsuccinique comprise entre 5 et 30%
➢ Diesters de l'acide adipique iusqu'à 10%.

11. Composition selon la revendication 10, **caractérisée en ce que en ce qu'**elle présente une température de cristallisation inférieure à -50°C.

12. Composition selon l'une des revendications 10 ou 11, **caractérisée en ce que** la solubilité de l'eau dans ladite composition est inférieure ou égale à 2.5% en poids à 23°C.

13. Composition selon l'une des revendications 10 à 12, **caractérisée en ce qu'**elle est obtenue par estérification de composés dinitriles correspondants.

14. Composition selon la revendication 13. **caractérisée en ce que** les composés dinitriles utilisés sont présents dans le mélange des dinitriles ramifiés séparés de l'adiponitrile dans le procédé de fabrication de l'adiponitrile par double hydrocyanation du butadiène.

15. Composition selon l'une des revendications 10 à 14, **caractérisée en ce que** les diesters sont obtenus par réaction d'un composé dinitrile avec un alcool choisi dans le groupe comprenant les alcools aliphatiques ramifiés ou non, cycliques ou non, pouvant comporter un noyau aromatique et pouvant comprendre de 1 à 20 atomes de carbone.

16. Composition selon la revendication 15, **caractérisée en ce que** l'alcool est choisi dans le groupe comprenant méthanol, propanol, isopropanol, alcool benzylique, éthanol, n-butanol, isobutanol, pentanols, cyclohexanol, hexanol, isooctanol, 2-éthyl hexanol.

17. Procédé de fabrication d'une composition diesters selon l'une des revendications 10 à 16, **caractérisé en ce qu'**il consiste à faire réagir un composé dinitrile avec un alcool en présence d'un acide minéral fort, puis à hydrolyser le milieu et à récupérer la composition de diesters.

18. Procédé de fabrication d'une composition diesters selon l'une des revendications 10 à 16, **caractérisé en ce qu'**il consiste à faire réagir en phase gaz, en présence d'un catalyseur solide, les dinitriles avec de l'eau et un alcool.

19. Procédé selon la revendication 18, **caractérisé en ce que** le catalyseur est un catalyseur solide acide.

20. Procédé selon la revendication 18, **caractérisé en ce que** le catalyseur est une alumine macroporeuse.

21. Procédé de fabrication d'une composition diesters selon l'une des revendications 10 à 16, **caractérisé en ce qu'**il consiste à faire réagir les dinitriles avec un composé basique pour obtenir des sels d'acides, à neutraliser ces sels avec un acide puis à estérifier les acides obtenus par réaction avec un alcool.

## Claims

1. Use as solvent or cosolvent of a composition comprising a mixture of diesters of ethylsuccinic acid and of methylglutaric acid.

2. Use according to Claim 1, **characterized in that** the composition comprises diesters of adipic acid.

3. Use according to either of Claims 1 and 2, **characterized in that** the concentration by weight of the various components of the composition is:
➢ diesters of methylglutaric acid of between 70 and 95%
➢ diesters of ethylsuccinic acid of between 5 and 30%
➢ diesters of adipic acid of between 0 and 10%.

4. Use according to one of the preceding claims, **characterized in that** the composition exhibits a crystallization temperature of less than -50°C.

5. Use according to one of the preceding claims, **characterized in that** the solubility of water in the said composition is less than or equal to 2.5% by weight at 23°C.

6. Use according to one of the preceding claims, **characterized in that** the composition is obtained by esterification of corresponding dinitrile compounds.

7. Use according to Claim 6, **characterized in that** the dinitrile compounds used are present in the mixture of the branched dinitriles which are separated from adiponitrile in the process for the manufacture of adiponitrile by double hydrocyanation of butadiene.

8. Use according to one of the preceding claims, **characterized in that** the diesters are obtained by reaction of a dinitrile compound with an alcohol chosen from the group consisting of branched or unbranched and cyclic or acyclic aliphatic alcohols which can comprise an aromatic ring and which can comprise from 1 to 20 carbon atoms.

9. Use according to Claim 8, **characterized in that** the alcohol is chosen from the group consisting of methanol, propanol, isopropanol, benzyl alcohol, ethanol, n-butanol, isobutanol, pentanols, cyclohexanol, hexanol, isooctanol and 2-ethylhexanol.

10. Composition comprising a mixture of diesters of ethylsuccinic acid, of methylglutaric acid and of adipic acid, **characterized in that** the concentration by weight of the various components is:
➢ diesters of methylglutaric acid of between 70 and 95%
➢ diesters of ethylsuccinic acid of between 5 and 30%
➢ diesters of adipic acid of up to 10%.

11. Composition according to Claim 10, **characterized in that** it exhibits a crystallization temperature of less than -50°C.

12. Composition according to either of Claims 10 and 11, **characterized in that** the solubility of water in the said composition is less than or equal to 2.5% by weight at 23°C.

13. Composition according to one of Claims 10 to 12, **characterized in that** it is obtained by esterification of corresponding dinitrile compounds.

14. Composition according to Claim 13, **characterized in that** the dinitrile compounds used are present in the mixture of the branched dinitriles which are separated from adiponitrile in the process for the manufacture of adiponitrile by double hydrocyanation of butadiene.

15. Composition according to one of Claims 10 to 14, **characterized in that** the diesters are obtained by reaction of a dinitrile compound with an alcohol chosen from the group consisting of branched or unbranched and cyclic or acyclic aliphatic alcohols which can comprise an aromatic ring and which can comprise from 1 to 20 carbon atoms.

16. Composition according to Claim 15, **characterized in that** the alcohol is chosen from the group consisting of methanol, propanol, isopropanol, benzyl alcohol, ethanol, n-butanol, isobutanol, pentanols, cyclohexanol, hexanol, isooctanol and 2-ethylhexanol.

17. Process for the manufacture of a diester composition according to one of Claims 10 to 16, **characterized in that** it consists in reacting a dinitrile compound with an alcohol in the presence of a strong inorganic acid, in then hydrolysing the medium and in recovering the diester composition.

18. Process for the manufacture of a diester composition according to one of Claims 10 to 16, **characterized in that** it consists in reacting the dinitriles with water and an alcohol in the gas phase in the presence of a solid catalyst.

19. Process according to Claim 18, **characterized in that** the catalyst is a solid acid catalyst.

20. Process according to Claim 18, **characterized in that** the catalyst is a macroporous alumina.

21. Process for the manufacture of a diester composition according to one of Claims 10 to 16, **characterized in that** it consists in reacting the dinitriles with a basic compound, in order to obtain acid salts, in neutralizing these salts with an acid and in then esterifying the acids obtained by reaction with an alcohol.

## Patentansprüche

1. Verwendung einer Zusammensetzung, die ein Gemisch von Ethylbernsteinsäure- und Methylglutarsäurediestern umfaßt, als Lösungsmittel oder Cosolvens.

2. Verwendung nach Anspruch 1, **dadurch gekennzeichnet, daß** die Zusammensetzung Adipinsäurediester umfaßt.

3. Verwendung nach Anspruch 1 oder 2, **dadurch gekennzeichnet, daß** sich die auf das Gewicht bezogene Konzentration der verschiedenen Bestandteile der Zusammensetzung auf folgende Werte beläuft:
➢ Methylglutarsäurediester zwischen 70 und 95%
➢ Ethylbernsteinsäurediester zwischen 5 und 30%
➢ Adipinsäurediester zwischen 0 und 10%.

4. Verwendung nach einem der vorhergehenden Ansprüche, **dadurch gekennzeichnet, daß** die Zusammensetzung eine Kristallisationstemperatur von weniger als -50°C aufweist.

5. Verwendung nach einem der vorhergehenden Ansprüche, **dadurch gekennzeichnet, daß** die Löslichkeit von Wasser in der Zusammensetzung kleiner gleich 2,5 Gew.-% bei 23°C beträgt.

6. Verwendung nach einem der vorhergehenden Ansprüche, **dadurch gekennzeichnet, daß** die Zusammensetzung durch Veresterung von entsprechenden Dinitrilverbindungen erhalten wird.

7. Verwendung nach Anspruch 6, **dadurch gekennzeichnet, daß** die verwendeten Dinitrilverbindungen in dem Gemisch der verzweigten Dinitrile, die beim Verfahren zur Herstellung von Adiponitril durch doppelte Hydrocyanierung von Butadien von Adiponitril abgetrennt werden, vorliegen.

8. Verwendung nach einem der vorhergehenden Ansprüche, **dadurch gekennzeichnet, daß** die Diester durch Umsetzung einer Dinitrilverbindung mit einem Alkohol aus der Gruppe bestehend aus verzweigten oder unverzweigten und cyclischen oder acyclischen Alkoholen, die einen aromatischen Kern enthalten und 1 bis 20 Kohlenstoffatome aufweisen können, erhalten werden.

9. Verwendung nach Anspruch 8, **dadurch gekennzeichnet, daß** der Alkohol aus der Gruppe bestehend aus Methanol, Propanol, Isopropanol, Benzylalkohol, Ethanol, n-Butanol, Isobutanol, Pentanolen, Cyclohexanol, Hexanol, Isooctanol und 2-Ethylhexanol ausgewählt ist.

10. Zusammensetzung, umfassend ein Gemisch von Ethylbernsteinsäure-, Methylglutarsäure- und Adipinsäurediestern, **dadurch gekennzeichnet, daß** sich die auf das Gewicht bezogene Konzentration ihrer verschiedenen Bestandteile auf folgende Werte beläuft:
➢ Methylglutarsäurediester zwischen 70 und 95%
➢ Ethylbernsteinsäurediester zwischen 5 und 30%
➢ Adipinsäurediester bis 10%.

11. Zusammensetzung nach Anspruch 10, **dadurch gekennzeichnet, daß** sie eine Kristallisationstemperatur von weniger als -50°C aufweist.

12. Zusammensetzung nach Anspruch 10 oder 11, **dadurch gekennzeichnet, daß** die Löslichkeit von Wasser in der Zusammensetzung kleiner gleich 2,5 Gew.-% bei 23°C beträgt.

13. Zusammensetzung nach einem der Ansprüche 10 bis 12, **dadurch gekennzeichnet, daß** sie durch Veresterung von entsprechenden Dinitrilverbindungen erhalten wird.

14. Zusammensetzung nach Anspruch 13, **dadurch gekennzeichnet, daß** die verwendeten Dinitrilverbindungen in dem Gemisch der verzweigten Dinitrile, die beim Verfahren zur Herstellung von Adiponitril durch doppelte Hydrocyanierung von Butadien von Adiponitril abgetrennt werden, vorliegen.

15. Zusammensetzung nach einem der Ansprüche 10 bis 14, **dadurch gekennzeichnet, daß** die Diester durch Umsetzung einer Dinitrilverbindung mit einem Alkohol aus der Gruppe bestehend aus verzweigten oder unverzweigten und cyclischen oder acyclischen Alkoholen, die einen aromatischen Kern enthalten und 1 bis 20 Kohlenstoffatome aufweisen können, erhalten werden.

16. Zusammensetzung nach Anspruch 15, **dadurch gekennzeichnet, daß** der Alkohol aus der Gruppe bestehend aus Methanol, Propanol, Isopropanol, Benzylalkohol, Ethanol, n-Butanol, Isobutanol, Pentanolen, Cyclohexanol, Hexanol, Isooctanol und 2-Ethylhexanol ausgewählt ist.

17. Verfahren zur Herstellung einer Diesterzusammensetzung nach einem der Ansprüche 10 bis 16, **dadurch gekennzeichnet, daß** man eine Dinitrilverbindung in Gegenwart einer starken anorganischen Säure mit einem Alkohol umsetzt, dann das Medium hydrolysiert und die Diesterzusammensetzung gewinnt.

18. Verfahren zur Herstellung einer Diesterzusammensetzung nach einem der Ansprüche 10 bis 16, **dadurch gekennzeichnet, daß** man die Dinitrile in der Gasphase in Gegenwart eines festen Katalysators mit Wasser und einem Alkohol umsetzt.

19. Verfahren nach Anspruch 18, **dadurch gekennzeichnet, daß** es sich bei dem Katalysator um einen festen sauren Katalysator handelt.

20. Verfahren nach Anspruch 18, **dadurch gekennzeichnet, daß** es sich bei dem Katalysator um makroporöses Aluminiumoxid handelt.

21. Verfahren zur Herstellung einer Diesterzusammensetzung nach einem der Ansprüche 10 bis 16, **dadurch gekennzeichnet, daß** man die Dinitrile mit einer basischen Verbindung zu Säuresalzen umsetzt, diese Salze mit einer Säure neutralisiert und die erhaltenen Säuren durch Umsetzung mit einem Alkohol verestert.
